(19) Europäisches Patentamt / European Patent Office / Office européen des brevets

(11) **EP 2 389 580 B1**

(12) **EUROPÄISCHE PATENTSCHRIFT**

(45) Veröffentlichungstag und Bekanntmachung des Hinweises auf die Patenterteilung:
**14.06.2017  Patentblatt 2017/24**

(21) Anmeldenummer: **09767965.8**

(22) Anmeldetag: **02.12.2009**

(51) Int Cl.:
**C07K 1/22** *(2006.01)*    **G01N 30/46** *(2006.01)*

(86) Internationale Anmeldenummer:
**PCT/EP2009/008575**

(87) Internationale Veröffentlichungsnummer:
**WO 2010/083859 (29.07.2010 Gazette 2010/30)**

(54) **VORRICHTUNG UND VERFAHREN FÜR DIE STOFFTRENNUNG**

DEVICE AND METHOD FOR MATERIAL SEPARATION

DISPOSITIF ET PROCÉDÉ POUR SÉPARER DES SUBSTANCES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **21.01.2009  DE 102009005479**

(43) Veröffentlichungstag der Anmeldung:
**30.11.2011  Patentblatt 2011/48**

(73) Patentinhaber: **Sartorius Stedim Biotech GmbH**
**37079 Göttingen (DE)**

(72) Erfinder:
- **GÓRAK, Andrzej**
  **58543 Witten (DE)**
- **KREIS, Peter**
  **44227 Dortmund (DE)**
- **VAN BEIJEREN BERGEN EN HENEGOUWEN, Peter**
  **50733 Köln (DE)**
- **FABER, René**
  **37077 Göttingen (DE)**
- **DEMMER, Wolfgang**
  **37077 Göttingen (DE)**
- **FRERICK, Constantin**
  **50733 Köln (DE)**

(74) Vertreter: **Müller-Boré & Partner**
**Patentanwälte PartG mbB**
**Friedenheimer Brücke 21**
**80639 München (DE)**

(56) Entgegenhaltungen:
**WO-A1-03/051483       WO-A1-2008/006780**
**US-A1- 2007 241 061**

- **SAITO K ET AL: "Radiation-induced graft polymerization is the key to develop high-performance functional materials for protein purification" RADIATION PHYSICS AND CHEMISTRY 199905 GB, Bd. 54, Nr. 5, Mai 1999 (1999-05), Seiten 517-525, XP004161328 ISSN: 0969-806X DOI: 10.1016/S0969-806X(98)00256-4**

EP 2 389 580 B1

**Beschreibung**

Die vorliegende Erfindung betrifft eine Vorrichtung zum Trennen bzw. Isolieren von Substanzen in bzw. aus einem Gemisch, wobei die Vorrichtung mindestens eine diffusiv betreibbare Chromatographiematrix und mindestens eine konvektiv betreibbare Chromatographiematrix umfasst. Darüber hinaus betrifft die vorliegende Erfindung sowohl die Verwendung dieser Vorrichtung als auch ein Verfahren zum Trennen und/oder Isolieren von Substanzen in bzw. aus einem Gemisch.

Physikalische Trennverfahren, bei denen die Stofftrennung durch Verteilung zwischen einer stationären und einer mobilen Phase geschieht, sind unter dem Begriff Chromatographie bekannt. Beispiele für solche chromatographischen Verfahren sind die Gel-Permeations-Chromatographie (GPC), Adsorptionschromatographie, Affinitätschromatographie, Ionenaustauschchromatographie und hydrophobe Interaktionschromatographie.

Zur Beurteilung der Leistungsfähigkeit solcher chromatographischen Trennsysteme werden üblicherweise die Produktivität, die dynamische Kapazität und die Lage der Durchbruchskurve im Chromatogramm für das jeweilige Chromatographiesystem herangezogen. Die Produktivität bezeichnet in diesem Zusammenhang die aus der Vorrichtung gewonnene Menge Produkt pro Zeiteinheit und pro Volumeneinheit des in dem jeweiligen Chromatographiesystem verwendeten Adsorbens. Dabei wird die dynamische Bindungskapazität als die Menge Substanz und damit das Volumen definiert, welches bis zu dem Zeitpunkt durch den Adsorber durchgelaufen ist, an dem die Konzentration der Substanz im Ablauf des Adsorbers einen gewissen Bruchteil der Konzentration der Substanz im Zulauf aufweist. Aus praktischen Gründen werden 5 bzw. 10 % der Zulaufkonzentration angegeben. Zur Aufnahme der kompletten Durchbruchskurve wird das die Substanz enthaltende Fluid so lange durch den Adsorber geleitet, bis dessen Kapazität erschöpft ist, er also vollständig mit Substanz beladen ist. Die Konzentration der Substanz im Auslauf ist dann genauso groß wie im Zulauf. Es resultiert ein sigmoider Kurvenverlauf. Durch Integration der Fläche unter der Kurve vom Startpunkt bis zum Punkt der maximalen Konzentration kann die nichtgebundene Menge, durch Integration der Fläche über der Kurve die gebundene Menge Substanz bestimmt werden. Das Verhältnis der beiden Flächen lässt Rückschlüsse auf die Güte des Adsobersystems zu.

Eine wirksame Maßnahme zur Verbesserung der Produktivität stellt hierbei beispielsweise die Erhöhung der linearen Flussrate dar. Dies hat jedoch den generellen Nachteil, dass der Durchbruch der Zielsubstanz wesentlich früher stattfindet und somit die Ausbeute regelmäßig stark abnimmt, insbesondere, wenn die aufzureinigende Produktmente pro Beladungszyklus konstant bleibt. Die Gel-Permeations-Chromatographie mit porösen, perlförmigen Teilchen (Beads) weist diesbezüglich zudem den Nachteil der Diffusionslimitierung des Massentransfers und dadurch einen starken Rückgang der dynamischen Bindungskapazität bei Erhöhung der linearen Flussrate auf. Gleichzeitig resultiert daraus eine flachere Durchbruchskurve. Dies ist besonders ausgeprägt, wenn der Adsorptionsmechanismus nicht wie bei Ionenaustauschern auf fernwirkenden elektrostatischen Wechselwirkungen beruht, sondern bei sogenannten Affinitätschromatographie-Prozessen auf der Kombination von nur auf kurzen Entfernungen (im molekularen Maßstab) wirksamen Kräften wie Coulombscher, Dipol- und/oder hydrophober Wechselwirkung beruht. Dabei müssen das zu bindende Zielmolekül (Target) und der spezifische Ligand in enge räumliche Nähe und richtige Ausrichtung kommen. Eine Erhöhung der linearen Flussrate hat hier ein extrem starkes Absinken der dynamischen Bindungskapazität zur Folge (vgl. Hahn et al., J. Chromatogr. A, 2005, 1093, Seiten 98-110).

Bei der Gel-Permeations-Chromatographie sind die Trennsäulen üblicherweise mit Beads eines porösen hochvernetzten Materials gefüllt. Durch dieses Material wird ein Fluid geleitet, welches Substanzen unterschiedlicher Molekülgröße enthält. Substanzen kleinerer Molekülgröße, bzw. mit einem kleineren hydrodynamischen Volumen, diffundieren in die Lösemittelgrenzschicht bzw. in die Poren des Gels und verbleiben dort, bis sie wieder aus der Lösungsmittelgrenzschicht bzw. den Poren herausdiffundieren. Bei größeren Molekülen finden diese Diffusionsvorgänge wesentlich langsamer statt, sodass eine Aufteilung der einzelnen Substanzen nach ihrer Molekülgröße erfolgt.

Die Gel-Permeations-Chromatographie ist daher ein sowohl in der chemischen als auch in der pharmazeutischen Entwicklung und Produktion häufig verwendetes Trennverfahren, insbesondere zur Isolation von Biomolekülen aus komplexen Gemischen, welche beispielsweise bei der Herstellung von Proteinen in Mikroorganismen oder bei der Isolation einzelner Bestandteile aus biologischen Fluiden, wie Blut, anfallen. Gelchromatographiesäulen weisen eine hohe dynamische Kapazität auf, können jedoch nur mit relativ geringen Flussraten betrieben werden.

Im Gegensatz zur Gel-Permeations-Chromatographie werden bei der (Membran-)Adsorptionschromatographie Komponenten eines Fluids, z.B. Einzelmoleküle, Assoziate oder Partikel, an der Oberfläche eines mit dem Fluid in Kontakt stehenden Festkörpers gebunden.

Ein zur Adsorption befähigter Festkörper wird "Adsorbens", die zu adsorbierende Komponente "Adsorbend" genannt. Die Adsorption kann technisch zur adsorptiven Stofftrennung eingesetzt werden, welche in "Adsorber" genannten Vorrichtungen ausgeführt wird. Der Adsorbend wird als "Zielsubstanz" bezeichnet, wenn seine Gewinnung aus dem Fluid beabsichtigt ist, und als "Kontaminante", wenn er aus diesem entfernt werden soll. Im ersten Fall muss die Adsorption umkehrbar sein, und der Adsorption folgt als zweiter Verfahrensschritt, unter geänderten Bedingungen (Zusammensetzung und/oder Temperatur) des Fluids, die Elution des Adsorbenden. Eine Zielsubstanz kann als einzige Komponente im Fluid enthalten sein, so dass die Stofftrennung in einer bloßen Anreicherung besteht, oder es liegen mehrere Komponenten vor, die getrennt werden sollen. In diesem Fall muss zumindest einer der Verfahrensschritte selektiv sein, also für die zu trennenden Komponenten in unterschiedlichem Ausmaß erfolgen. Die im Gleichgewicht gebundene Masse des Adsorbenden, bezogen auf die Masseneinheit des Adsorbens wird "statische Bindungskapazität" genannt. Deren Abhängigkeit von der Konzentration des Adsorbenden im Fluid wird durch die Adsorptionsisotherme beschrieben. Mitentscheidend für die Kapazität eines Adsorbers ist seine spezifische Oberfläche, weshalb Adsorbentien vorzugsweise eine hohe Porosität aufweisen. Man unterscheidet zwischen äußerer spezifischer Oberfläche, d.h. dem geometrischen Oberflächen-Massenverhältnis, und der inneren spezifischen Oberfläche, d.h. dem Porenoberflächen-Massenverhältnis. Voraussetzung für die Bindungsverfügbarkeit der inneren Oberfläche ist ihre sterische Zugänglichkeit für den Adsorbenden, also dessen Ausschlußgrenze, welche für die Chromatographie beispielsweise durch die Masse globulärer Proteine, die in die Poren gerade nicht mehr eindringen können, charakterisiert wird.

Die Fähigkeit zur Adsorption kann einem Feststoff inhärent sein, wie im Falle von Aktivkohle und Hydroxylapatit, oder durch adsorptive Modifizierung eines Basismaterials, d.h. eines vorzugsweise adsorptiv inerten Feststoffes geeigneter Morphologie, erreicht werden, die darin besteht, dass an oberflächlichen Ankergruppen des Basismaterials chemische Einheiten kovalent gebunden werden, welche als Liganden bezeichnet werden und welche vorzugsweise zur selektiven Bindung befähigt sind.

Der Vorteil von Membranadsorbern gegenüber gepackten Chromatographiesäulen liegt in ihrer Eignung, mit wesentlich höheren Flussraten betrieben werden zu können. Jedoch verfügen Membranadsorber im Gegenzug über eine geringere dynamische Kapazität als beispielsweise Gel-Permeations-Chromatographiesäulen. Aufgrund dieser geringeren Kapazität von Membranadsorbern sind sie nicht für die Anwendung hochkonzentrierter Lösungen geeignet. Um diesen Nachteil zu überwinden, wurden bereits Versuche unternommen, mehrere dieser Membranadsorbereinheiten in Reihe zu schalten, um somit die Vorteile einer hohen Flussrate mit denen einer hohen Kapazität zu vereinen.

In diesem Zusammenhang beschreiben die Druckschriften US 6,294,090 B1 und DE 197 11 083 A1 eine Serienschaltung von Adsorbermodulen zur Verbesserung der dynamischen Kapazität von großen Modulen im konvektiven Betrieb. Der ungleichmäßige Durchbruch der Zielsubstanz soll in einer solchen Anordnung durch die nachfolgenden Adsorberstufen kompensiert werden. Hierzu wird eine äußerst stark abgereicherte Lösung der Zielsubstanz eingesetzt und eine praktisch vollständige Vermischung des Durchlaufes und damit der Zielsubstanz ausgenutzt, so dass eine kleinere Adsorberstufe verwendet werden kann. Durch entsprechende Geometrie der hintereinander geschalteten Stufen ist die hydraulische Permeabilität in allen Stufen gleich. Eine solche Verschaltung unterschiedlich dimensionierter chromatographischer Einheiten ist technisch jedoch äußerst aufwendig und mit Hinblick auf den geringen Effekt nicht sinnvoll.

In der Druckschrift WO 03/051483 A1 wird ein chromatographisches Verfahren zur Isolierung und Reinigung eines Polynukleotids aus einem Fluid beschrieben, welches aus einer Kombination von zwei Schritten besteht. Jeder Schritt soll auf einem anderen chromatographischen Prinzip basieren, welches ausgewählt wird aus: a) hydrophober Interaktion, b) polarer Interaktion und c) Ionenaustausch). Mindestens einer der beiden Schritte besteht in der Verwendung eines porösen monolithischen Bettes (Monolith) als einer konvektiv betreibbaren monolithischen Chromatographiematrix. Für den Fall, dass nur in einem der beiden Schritte ein Monolith verwendet wird, erfolgt die Durchführung des anderen Schrittes durch Verwendung einer diffusiv betreibbareren Chromatographiematrix.

In der Druckschrift US 2007/0241061 A1 wird eine mikromechanische Vorrichtung zur Abtrennung einer Substanz aus einer Flüssigkeit beschrieben, die aus zwei hintereinander geschalteten Betten mit einem porösen Material bestehen kann. Die beiden Betten werden über eine poröse Filtrationsmembran kommunizierend verbunden, welche ein Vermischen der Partikel zwischen den Betten verhindern soll. Eines der zwei porösen Betten kann ein poröser Monolith sein. Das vorgeschaltete poröse Bett ist unwirksam gegenüber der abzutrennenden Substanz. Es soll Schadstoffe zurückhalten.

Nachteilig ist, dass im Monolithen unregelmäßige poröse Strukturen auftreten können, die eine hohe Passage der zu isolierenden und zu reinigenden Substanz führen und seine komplizierte Herstellung. Der vorliegenden Erfindung liegt somit die Aufgabe zugrunde, eine Vorrichtung bereitzustellen, welche die vorstehend genannten Nachteile überwindet und zu einer verbesserten Trennleistung bei hoher Produktivität führt.

Diese Aufgabe wird durch die in den Ansprüchen charakterisierten Ausführungsformen gelöst.

Insbesondere wird eine Vorrichtung zum Trennen bzw. Isolieren von Substanzen in bzw. aus einem Gemisch in einem Fluid bereitgestellt, welche mindestens eine diffusiv betreibbare Chromatographiematrix und mindestens eine nachgeschaltete, konvektiv betreibbare, flächenförmig ausgestaltete Chromatographiematrix umfasst, wobei die konvektiv betreibbare Chromatographiematrix der diffusiv betreibbaren Chromatographiematrix so nachgeschaltet ist, dass das die diffusiv betreibbare Chromatographiematrix verlassende Fluid durch die konvektiv betreibbare Chromatographiematrix leitbar ist, wobei die diffusiv betreibbare Chromatographiematrix eine Gelchromatographiematrix ist, und wobei die konvektiv betreibbare Chromatographiematrix ein Membranadsorber mit mindestens einer Membran ist.

Die erfindungsgemäße Kombination einer diffusiv betreibbaren Chromatographiematrix mit einer nachgeschalteten konvektiv betreibbaren Chromatographiematrix wird im Folgenden mit "serieller (Ver-)Schaltung" bezeichnet. Der Ausdruck "Trennen bzw. Isolieren", wie hierin verwendet, unterliegt keiner speziellen Einschränkung und betrifft jeden Vorgang, der geeignet ist, eine Substanz aus einem Gemisch zu entfernen. Hierbei bedeutet der Ausdruck "Trennen bzw. Isolieren" beispielsweise, dass eine gewünschte Zielsubstanz aus einem Gemisch, welches in einem Fluid vorliegt, im Sinne einer Reinigung isoliert wird oder dass eine Verunreinigung entfernt wird, um das Gemisch von dieser Verunreinigung zu befreien. Auch beinhaltet dieser Ausdruck keinerlei Einschränkung bezüglich der zu trennenden Mengen und betrifft sowohl die Trennung analytischer Substanzmengen als auch die präparative Aufreinigung größerer Substanzmengen, die beispielsweise im Rahmen einer Produktion anfallen können.

Darüber hinaus betrifft der Ausdruck "Gemisch" jedes Gemisch, welches mehr als eine Substanz enthält. Gemäß der vorliegenden Erfindung kann ein solches Gemisch eine Komponente in großem Überschuss enthalten, wobei die restlichen Komponenten des Gemisches in kleinen oder kleinsten Mengen vorhanden sind. Der Begriff "Gemisch" beinhaltet jedoch auch solche Gemische, welche mehrere Komponenten enthalten, die in mittlerer Konzentration vorliegen.

Der Ausdruck "diffusiv betreibbare Chromatographiematrix", wie hierin verwendet, beinhaltet allgemein jedwede, aus Partikeln bestehende Matrix, die im wesentlichen eine Diffusionslimitierung des Massentransfers aufweist, die darin besteht, daß die Geschwindigkeit des Adsorptions- und Desorptionsprozesses von der Diffusionsgeschwindigkeit der Substanz(en) in und aus den Partikeln heraus durch die Diffusionskoeffizienten der Substanz(en), welche sehr stark von der Größe, bzw. dem Molekulargewicht der Substanzen abhängen, bestimmt wird.

Gemäß der vorliegenden Erfindung ist die diffusiv betreibbare Chromatographiematrix ein poröses, partikuläres Adsorbens.

Beispiele für bevorzugte diffusiv betreibbare Chromatographiematrizen sind poröse, partikuläre Adsorbentien, deren Partikel regulär oder irregulär geformt sein können und die als Schüttung in einer mit Ein- und Auslaß versehenen Säule vorliegen können. Besonders bevorzugt weisen die Matrizen ionenaustauschende Gruppen auf.

Diffusiv betreibbare Chromatographiematrizen mit regulären, z. B. sphärisch geformten, Partikeln sind beispielsweise quervernetzte Agarosen, wie z. B. DEAE Sepharose® CL-6B (mit DEAE Diethylaminoethyl-Ligand), Q-Sepharose® HP (mit Q Trimethylamin-Ligand), Q-Sepharose® FF, S-Sepharose® Fast Flow (mit S Sulfonsäureligand).

Beispielsweise können allgemein weiterhin "Controlled pore glass"-Matrizen oder periförmige Cellulosen, wie z. B. DEAE- Sephacel®; DEAE Cellufine®, CM und C-200 Cellufine®, zum Einsatz kommen.

Weitere diffusiv betreibbare Chromatographiematrizen mit sphärisch geformten Partikeln sind auf Dextran basierende Chromatographiegele, wie beispielsweise DEAE Sephadex®, QAE-Sephadex® (mit QAE [(CH$_3$CH(OH)CH$_2$)(C$_2$H$_5$)$_2$]N$^+$CH$_2$CH$_2$O-Ligand), SP Sephadex® (mit SP Propansulfonsäure-Ligand).

Weitere diffusiv betreibbare Chromatographiematrizen, deren Partikel sphärisch geformt sind, sind auf Polymer-Kompositen basierende Matrizen, wie beispielsweise Fractogel® EMD, TMAE-650, DEAE-Toyopearl® 650S, DEAE-Trisacryl® M, DEAE-Spherodex® M, Spherosil® M, Macroprep® Q, Q Hyper® D, M Porös® 50 HQ sowie in keramischen Mikrokapseln vorliegende Polyacrylamidgele.

Diffusiv betreibbare Chromatographiematrizen mit irregulär geformten Partikeln sind beispielsweise Matricen, ausgewählt aus der Gruppe von Aktivkohle, Hydroxylapatit, Silica und Kieselgur.

Gemäß der vorliegenden Erfindung wird als diffusiv betreibbare Chromatographiematrix eine Gelchromatographiematrix eingesetzt, wobei Sepharose® FF des Typs Q (mit Q Trimethylaminligand) der Firma GE Healthcare Life Sciences in einer HiPrep®-Säule der Firma GE Healthcare Life Sciences oder als Packung in einer 20-ml-XK-16-Säule der Firma GE Healthcare Life Sciences am meisten bevorzugt ist (nachfolgend "Q Sepharose® FF HiPrep®" genannt). In einer weiteren bevorzugten Ausführungsform wird Sepharose® XL des Typs Q (Q Trimethylaminligand) der Firma GE Healthcare Life Sciences als Packung in einer 20-ml-XK-16-Säule der Firma GE Healthcare Life Sciences eingesetzt.

Der Ausdruck "konvektiv betreibbare Chromatographiematrix", wie hierin verwendet, beinhaltet allgemein jedwede Matrix, bei welcher durch Anlegen einer hydraulischen Druckdifferenz zwischen Zulauf und Ablauf der Matrix eine Durchströmung der Matrix erzwungen wird, wodurch ein im wesentlichen konvektiver Transport der Substanz(en) in die Matrix hinein bzw. aus der Matrix heraus erreicht wird, der bei hohem Durchfluß sehr rasch erfolgt. Der geschwindigkeitsbestimmende Schritt wird dann praktisch nur durch die Assoziationskonstante(n) zwischen der Matrix und der/den Substanz(en) bestimmt.

Gemäß der vorliegenden Erfindung ist die konvektiv betreibbare Chromatographiematrix flächenförmig ausgestaltet.

Beispiele für bevorzugte, erfindungsgemäß konvektiv betreibbare Chromatographiematrizen, sind solche Chromatographiematrizen, die flächenförmig ausgestaltet sind und Gebilde darstellen, bei denen die Ausdehnung in der x- und y-Achse im kartesianischen, dreidimensionalen Koordinatensystem die Ausdehnug in der z-Achse um ein Vielfaches übersteigt. Diese flächigen Gebilde können nun ihrerseits in Form von Flachmodulen, Stapelmodulen, Wickelmodulen oder Zylindern konvektiv betreibbar sein. Flächige, konvektiv betreibbare Chromatographiematrizen sind beispielsweise Filter, Membranen, Vliese, Gewebe oder Kombinationen davon.

Allgemein können konvektiv betreibbare Chromatographiematrix aus der Gruppe von Filtern, Membranen, Vliesen, Geweben oder Kombinationen davon ausgewählt sein.

Unter "Filtern" sollen flächige Gebilde verstanden werden, die eine Abtrennung von Substanzen aus flüssigen oder gasförmigen Fluiden erlauben, wobei durch die Siebwirkung die Partikel, welche größer als die nominelle Abscheiderate des Filters sind, zurückgehalten werden. Diese Filter können funktionelle Gruppen auf ihrer Oberfläche tragen.

Allgemein können die konvektiv betreibbaren Chromatographiematrizen monolithisch sein, d.h. sie sind nicht aus einer Vielzahl von Partikeln aufgebaut, sondern stellen einstückige, zusammenhängende und poröse Monolithen dar, welche konvektiv durchströmbar sind. Kommerziell erhältliche, monolithische Matrizen werden von den Firmen BIA, Ljubliana, Slowenien, (CIM-Disks), und Biorad Hercules Ca., USA, (UNO Monolith) vertrieben.

Gemäß der vorliegenden Erfindung ist die konvektiv betreibbare Chromatographiematrix ein Membranadsorber mit mindestens einer Membran.

Als konvektiv betreibbare Chromatographiematrizen werden erfindungsgemäß bevorzugt Membranadsorber verwendet, welche mindestens eine mikroporöse Membran umfassen, die optional auf ihrer inneren und äußeren Oberfläche funktionelle Gruppen trägt, welche mit den Substanzen in physikalische und/oder chemische Wechselwirkung treten. Unter "chemischer Wechselwirkung" soll nachfolgend jedwede kovalente, ionogene und/oder auf elektrostatischen oder hydrophoben (z. B. Van-der-Waal-Wechselwirkung) Interaktionen beruhende Bindungswechselwirkung zwischen den funktionellen Gruppen und den Adsorbenden verstanden werden.

Der hierin verwendete Ausdruck "nachgeschaltet" betrifft lediglich die Tatsache, dass die konvektiv betreibbare Chromatographiematrix in Fliessrichtung hinter der diffusiv betreibbaren Chromatographiematrix angeordnet ist und ist darüber hinaus nicht als einschränkend zu verstehen.

Gemäß einer besonders bevorzugten Ausführungsform der vorliegenden Erfindung ist das die diffusiv betreibbare Chromatographiematrix verlassende Fluid vollständig durch die konvektiv betreibbare Chromatographiematrix leitbar.

Gemäß einer spezifischen, besonders bevorzugten Ausführungsform der vorliegenden Erfindung ist die konvektiv betreibbare Chromatographiematrix unmittelbar mit der diffusiv betreibbaren Chromatographiematrix verbunden, so dass das gesamte Fluid, welches aus der diffusiv betreibbaren Chromatographiematrix austritt, direkt und vollständig durch die konvektiv betreibbare Chromatographiematrix leitbar ist.

Gemäß einer besonders bevorzugten Ausführungsform der vorliegenden Erfindung liegt die Kombination von diffusiv betreibbarer Chromatographiematrix und konvektiv betreibbarer Chromatograhiematrix in einer kompakten bzw. einheitlichen Baugruppe vor, sodass eine einfache Handhabung, beispielsweise zur Wartung oder zum Austausch der Vorrichtung, unproblematisch erfolgen kann.

Der erfindungsgemäß verwendete Begriff "Chromatographiematrix" umfasst jedes Material, an dessen Oberfläche mindestens eine Komponente eines Fluids, welches mit dem Material in Kontakt steht, gebunden werden kann. Die Fähigkeit zur Absorption im Sinne der vorliegenden Erfindung kann dem Material der "Chromatographiematrix" inhärent sein, wie beispielsweise im Falle von Aktivkohle oder Hydroxylapatit, sie kann jedoch auch durch Modifizierung eines Trägermaterials mit einem oder mehreren Liganden erzielt werden, wobei die diffusiv betreibbare und/oder die konvektiv betreibbare Chromatographiematrix mindestens einen Liganden umfaßt, der über mindestens eine chemische und/oder physikalische Wechselwirkung mit mindestens einer Substanz in dem Gemisch interagiert.

Beispiele für derart verwendbare Liganden sind Liganden, die über mindestens eine chemische und/oder physikalische Wechselwirkung mit Adsorbenden interagieren, insbesondere Ionenaustauscher, salztolerante Liganden, Chelatbildner, thiophile oder hydrophobe Liganden mit Substituenten verschiedener Kettenlängen und Konfigurationen, "Reversed-Phase"-Liganden, reaktive und andere Farbstoffe, anorganische Moleküle und Ionen sowie deren organische und anorganische Verbindungen, Affinitätsliganden, darunter niedermolekulare ungeladene oder geladene organische Moleküle, Aminosäuren und deren Analoga, Coenzyme, Cofaktoren und deren Analoga, Substrate und deren Analoga, endokrine und exokrine Substanzen wie Hormone und hormonähnlich wirkende Effektoren sowie deren Analoga, Enzym-Substrate, -Inhibitoren und deren Analoga, Fettsäuren, Fettsäurederivate, konjugierte Fettsäuren und deren Analoga, Nukleinsäuren wie DNS und deren Analoga und Derivate, RNS und deren Analoga und Derivate, Monomere und deren Analoga und Derivate, Oligo- bis Polymere und deren Analoga und Derivate, hochmolekulare Kohlenhydrate, linear oder verzweigt, unsubstituiert oder substituiert, Glycokonjugate, wie Heparin, Amylose, Zellulose, Chitin, Chitosan, Monomere und Oligomere sowie deren Derivate und Analoga sowie Lignin und dessen Derivate und Analoga.

Weitere Beispiele sind hochmolekulare Liganden wie Proteine und ihre Oligomere, Multimere, Untereinheiten sowie Teile davon, Peptide, Polypeptide, deren Analoga und Derivate, Lectine, Antikörper und Teile davon, Fusionsproteine, Haptene, Enzyme und Untereinheiten sowie Teile davon, Strukturproteine, Rezeptoren und Effektoren sowie Teile davon, Viren und Teile davon, Xenobiotika, Pharmazeutika und pharmazeutische Wirkstoffe, Alkaloide, Antibiotika, Biomimetika und Katalysatoren.

Eine bevorzugte Ausführungsform der vorliegenden Erfindung betrifft eine wie vorstehend definierte Vorrichtung, wobei wenigstens die konvektiv betreibbare Chromatographiematrix einen oder mehrere Ligand(en) umfasst, welcher bzw. welche über mindestens eine chemische und/oder physikalische Wechselwirkung mit Adsorbenden interagiert bzw. interagieren und welcher bzw. welche aus der Gruppe umfassend Ionenaustauscher, salztolerante Liganden, Chelatbildner, thiophile oder hydrophobe Liganden verschiedener Kettenlängen und Konfigurationen, "Reversed-Phase"-Liganden, reaktive und andere Farbstoffe, anorganische Moleküle und Ionen, deren organische und anorganische Verbindungen, Affinitätsliganden, hochmolekulare Liganden, Enzyme und Untereinheiten sowie Teile davon, Strukturproteine, Rezeptoren und Effektoren sowie Teile davon, Viren und Teile davon, Xenobiotika, Pharmazeutika und pharmazeutische Wirkstoffen, Alkaloide, Antibiotika, Biomimetika und Katalysatoren ausgewählt ist bzw. sind.

Besonders bevorzugte Liganden, die an die diffusiv betreibbare und/oder konvektiv betreibbare Chromatographiematrix gebunden sind, sind Trimethylamin-, N,N-Diethyl-N-(2-hydroxy-1-propyl)-ammoniummethyl-, Diethylaminoethyl-, 2,2'-Iminodiethanol-, Carboxymethyl-, Sulfopropyl- und Sulfomethyl-Liganden.

Bei einer bevorzugten Ausführungsform der erfindungsgemäßen Vorrichtung umfasst die diffusiv betreibbare und die konvektiv betreibbare Chromatographiematrix jeweils mindestens einen Liganden, wobei diese Liganden über dieselbe Art von Bindungswechselwirkung mit den Substanzen in dem Gemisch interagieren (z. B. durch Kationen- oder Anionenaustausch). In einer besonders bevorzugten Ausführungsform tragen beide Chromatographiematrizen kationenaustauschende Liganden, wie z. B. Sulfsonsäure- oder Sulfopropylgruppen.

Bei einer weiteren bevorzugten Ausführungsform der wie vorstehend definierten Vorrichtung umfassen sowohl die diffusiv betreibbare als auch die konvektiv betreibbare Chromatographiematrix mindestens einen gleichen Liganden.

Bei einer besonders bevorzugten Ausführungsform ist sowohl an die diffusiv betreibbare als auch an die konvektiv betreibbare Chromatographiematrix Trimethylamin als Ligand gebunden.

Eine weitere Ausführungsform der vorliegenden Erfindung betrifft eine wie vorstehend definierte Vorrichtung, wobei die nachgeschaltete konvektiv betreibbare Chromatographiematrix eine geringere statische Bindungskapazität als die vorgelagerte, diffusiv betreibbare Chromatographiematrix aufweist.

Gemäß einer weiteren Ausführungsform der vorliegenden Erfindung ist die nachgeschaltete, konvektiv betreibbare Chromatographiematrix eine Membranadsorbereinheit, welche weniger als 50 %, vorzugsweise weniger als 20 %, der statischen Bindungskapazität der vorgelagerten, diffusiv betreibbaren Chromatographiematrix, welche eine Gelchromatographiematrix umfaßt, aufweist.

Ein weiterer Aspekt der vorliegenden Erfindung betrifft ein Verfahren zum Trennen bzw. Isolieren von Substanzen in bzw. aus einem Gemisch, umfassend die Schritte (a) gegebenenfalls des Lösens des Gemisches in einem geeigneten Lösungsmittel oder Lösungsmittelgemisch, um ein Fluid zu erhalten, (b) des Hindurchleitens des Fluids durch die vorstehend definierte Vorrichtung und (c) des Sammelns des hindurchgeleiteten Fluids.

Der Begriff "Lösen" betrifft jeden Vorgang, bei dem eine Substanz in einem Lösungsmittel gelöst wird und unterliegt darüber hinaus keinen weiteren Einschränkungen. Dabei kann das "Lösungsmittel" ein Reinstoff oder ein Gemisch aus verschiedenen Lösungsmitteln sein. Gemäß der vorliegenden Erfindung kann das Lösungsmittel darüber hinaus weitere Verbindungen, wie beispielsweise Salze, Basen oder Säuren enthalten. Desweiteren kann das Lösungsmittel Substanzen enthalten, welche zur Identifikation von Stoffen oder zur Kalibrierung dienen.

Gemäß der vorliegenden Erfindung liegt die Flussrate, mit welcher das Fluid durch die vorstehend definierte Vorrichtung hindurchgeleitet wird, in einem Bereich von 0,01 ml/min bis 1000 ml/min, vorzugsweise in einem Bereich von 1 ml/min bis 200 ml/min, besonders bevorzugt in einem Bereich von 2 ml/min bis 100 ml/min und am meisten bevorzugt im Bereich von 2,5 ml/min bis 50 ml/min.

Gemäß der vorliegenden Erfindung liegt bzw. liegen die jeweilige(n) Anfangskonzentration(en) der zu trennenden bzw. zu isolierenden Zielsubstanz(en) in dem Fluid in einem Bereich von 0,001 mg/ml bis 1000 mg/ml, vorzugsweise in einem Bereich von 0,01 mg/ml bis 50 mg/ml, besonders bevorzugt in einem Bereich von 0,1 mg/ml bis 20 mg/ml und am meisten bevorzugt in einem Bereich von 1 mg/ml bis 10 mg/ml, bezogen auf die Einzelsubstanz.

Eine weitere Ausführungsform betrifft ein wie vorstehend definiertes Verfahren, wobei das Gemisch eine Lösung aus einer Synthese, ein biomolekülhaltiges Fluid oder ein biopartikelhaltiges Fluid ist.

Unter "biomolekülhaltigem Fluid" soll hierbei ein Fluid verstanden werden, welches Substanzen, ausgewählt aus der Gruppe der Aminosäuren und deren Analoga, Coenzyme, Cofaktoren und deren Analoga, der Endokrine und der exokrinen Substanzen, wie Hormone und hormonähnlich wirkende Effektoren, und deren Analoga, Enzyme und Untereinheiten sowie Teilen davon, Enzym-Substrate, Enzym-Inhibitoren und deren Analoga, Fettsäuren, Fettsäurederivate, konjugierten Fettsäuren und deren Analoga, der Desoxy- und Ribonukleinsäuren und deren Analoga und Derivate, ein- bis mehrsträngigen Plasmide, Cosmide und anderer Konstrukte, Kohlenhydrate und Glycokonjugate, Proteine und ihrer Oligomere, Multimere, Untereinheiten sowie Teilen davon, Peptide, Polypeptide, deren Analoga und Derivate, Lektine, Antikörper und Teilen davon, Fusionsproteine, Haptene, Strukturproteine, Rezeptoren und Effektoren sowie Teilen davon, Xenobiotika, Pharmazeutika, Alkaloide, Antibiotika, Biomimetika, Allergene oder Kombinationen davon, enthält.

Unter "biopartikelhaltigem Fluid" soll hierbei ein Fluid verstanden werden, welches Partikel, ausgewählt aus der Gruppe der Viren und Teilen davon, Prionen und Teilen davon, Mikroorganismen, Prokaryonten, Protozoen, Hefen, Pilze, Eukaryonten tierischen und pflanzlichen Ursprungs, Zellwandbestandteile von Prokaryonten, Protozoen, Hefen, Pilze, Eukaryonten tierischen und pflanzlichen Ursprungs und Teilen davon, biologischen Membranen und Teilen davon, subzellulären, biologischen Membranen und Teilen davon oder Kombinationen davon, enthält.

Eine bevorzugte Ausführungsform der vorliegenden Erfindung betrifft das vorstehend definierte Verfahren, wobei das Gemisch Blutplasma und die zu isolierende Substanz Serumalbumin ist.

Ein weiterer Aspekt der vorliegenden Erfindung betrifft die Verwendung der vorstehend definierten Vorrichtung zum Trennen bzw. Isolieren mindestens einer Zielsubstanz in bzw. aus einem Gemisch.

Eine solche Verwendung ist nicht auf einen bestimmten technischen Bereich begrenzt und umfasst alle Anwendungen, in welchen Substanzen aus einem Gemisch getrennt oder isoliert werden müssen. Beispiele solcher Bereiche sind die chemische, pharmazeutische, medizinische oder biologische Forschung oder Produktion.

Die Figuren zeigen:

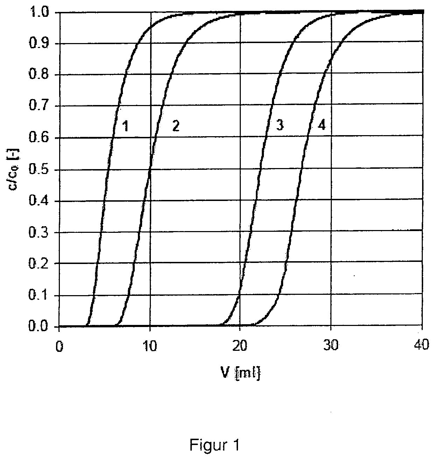

Figur 1 zeigt Durchbruchskurven der nichtbindenden Substanz Aceton bei einer Flussrate von 10 ml/min zur Bestimmung des Totvolumens der verschiedenen folgenden Konfigurationen:

Graph 1 zeigt die Durchbruchskurve der Chromatographie-Anlage "ÄKTA Prime plus" des Herstellers General Electrics (GE) Healthcare (im Weiteren "ÄKTA" genannt) ohne Säule und Adsorber.
Graph 2 zeigt die Durchbruchskurve der Chromatographie-Anlage ÄKTA in Verbindung mit dem Membranadsorbermodul Sartobind® Q Nano (30-lagig, 8 mm Betthöhe, 3 ml Membranvolumen) des Herstellers Sartorius Stedim Biotech GmbH (im Weiteren "Sartobind® Q Nano" oder "Nano" genannt).
Graph 3 zeigt die Durchbruchskurve der Kombination von ÄKTA, einer Q Sepharose® FF HiPrep®-Säule (im Weiteren "Q Sepharose® FF HiPrep®" oder "HiPrep®" genannt).
Graph 4 zeigt die Durchbruchskurve der Kombination von ÄKTA, Q Sepharose® FF HiPrep® und Sartobind® Q Nano (Serielle (Ver-)Schaltung).

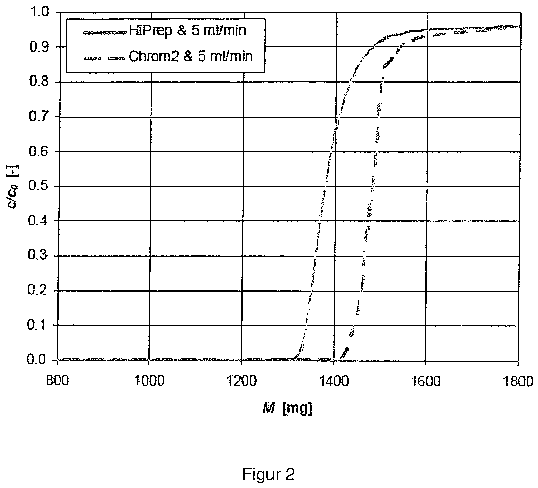

Figur 2 zeigt einen Vergleich der Durchbruchskurven für eine Q Sepharose® FF HiPrep®-Säule ("HiPrep") und eine serielle Verschaltung ("Chrom2", d.h. eine Kombination der Q Sepharose® FF HiPrep®-Säule und Sartobind® Q Nano) bei einer Flussrate von 5 ml/min. Die Konzentration des Proteins (bovines Serumalbumin) betrug in beiden Fällen 2 g/L.

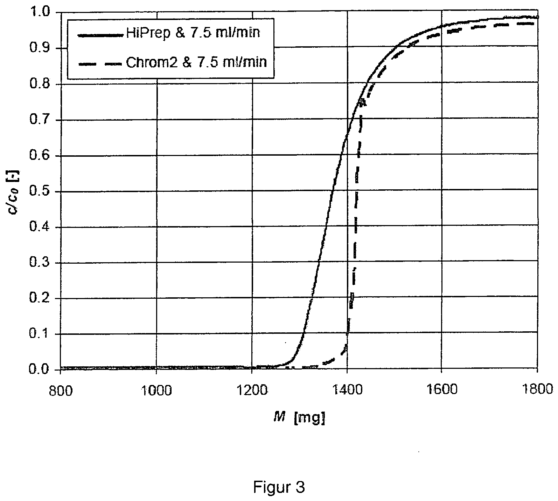

Figur 3 zeigt einen Vergleich der Durchbruchskurven für eine Q Sepharose® FF HiPrep®-Säule ("HiPrep") und eine serielle Verschaltung ("Chrom2", d.h. eine Kombination der Q Sepharose® FF HiPrep®-Säule und Sartobind® Q Nano) bei einer Flussrate von 7,5 ml/min. Die Konzentration des Proteins (bovines Serumalbumin) betrug in beiden Fällen 2 g/L.

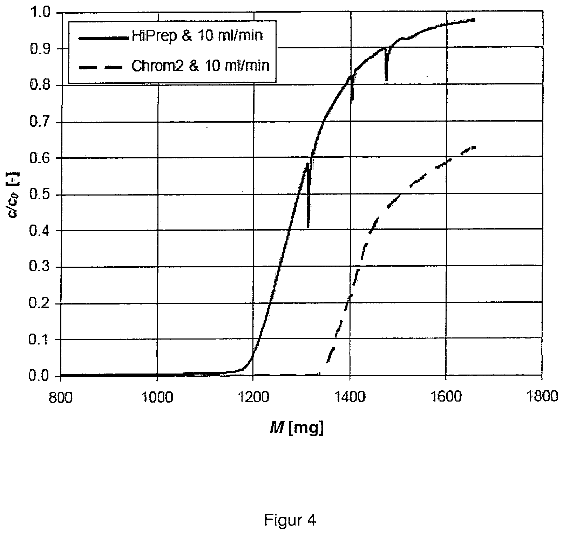

Figur 4 zeigt einen Vergleich der Durchbruchskurven für eine Q Sepharose® FF HiPrep®-Säule ("HiPrep") und eine serielle Verschaltung ("Chrom2", d.h. eine Kombination der Q Sepharose® FF HiPrep®-Säule und Sartobind® Q Nano) bei einer Flussrate von 10 ml/min. Die Konzentration des Proteins (bovines Serumalbumin) betrug in beiden Fällen 2 g/L.

Die erfindungsgemäße chromatographische Vorrichtung, welche eine diffusiv betreibbare Chromatographiematrix und eine nachgeschaltete, konvektiv betreibbare, flächenförmig ausgestaltete Chromatographiematrix umfasst, ermöglicht das Trennen bzw. Isolieren einer Zielsubstanz in bzw. aus einem Gemisch auf überraschend effektive Weise. Die Kombination einer Gelchromatographiesäule mit einer Membranadsorbereinheit führt besonders vorteilhaft zu einer verbesserten Produktivität ohne Verlust der dynamischen Bindungskapazität und zeichnet sich durch eine überraschend steile Durchbruchskurve der Zielsubstanz aus. Hierdurch können überraschend die Vorzüge der Säulenchromatographie mit denen der (Membran- )Adsorptionschromatographie verbunden werden. Die erfindungsgemäße Vorrichtung und deren Verwendung ermöglichen das effektive Trennen von Substanzen, welche beispielsweise durch Züchten von Mikroorganismen hergestellt werden, oder welche aus biologischen Fluiden, wie Blut, isoliert werden. Diese erfindungsgemäßen Effekte sind daher überraschend, weil eine Gelchromatographiematrix und eine Membranadsorbermatrix grundsätzlich unterschiedlichen Betriebsparametern (statische und dynamische Kapazität, Flussraten, etc) unterliegen, so dass eine unmittelbare Kombination beider in einer Baugruppe besonders fern liegt. Durch die Kombination einer Gelchromatographiematrix mit einer nachgeschalteten Membranadsorbereinheit in der erfindungsgemäßen Vorrichtung ist die Produktivität für die abzutrennenden bzw. aufzureinigenden Zielsubstanzen ohne Verlust der dynamischen Bindungskapazität um mindestens 30 % höher als die Produktivität der Gelchromatographiematrix.

BEISPIELE

Die vorliegende Erfindung wird im Folgenden durch die Beispiele näher erläutert, ohne jedoch durch diese in irgendeiner Weise eingeschränkt zu sein.

Beispiel 1: Verbesserung der dynamischen Bindungskapazität durch Verwendung von Chromatographiesäule und Membran-Adsorber.

Eine Q Sepharose® FF HiPrep®-Säule wurde durch Packen einer 20-ml-XK 16 Säule der Fa General Electrics (GE) Healthcare Life Sciences mit 20 ml einer Sepharose® FF des Typs Q (starker Anionenaustauscher), Bestellnummer 17-507201, Lot Nr.: 302853, bereitgestellt. Der Ligand ist Trimethylamin, wobei R die Matrix bezeichnet ($R-N^+-(CH_3)_3$).
Als zweite Stufe wurde ein Membranstapel von Sartobind® Q Nano, 30-lagig, 8 mm Betthöhe, mit einem Membranvolumen von 3 ml der Fa. Sartorius Stedim Biotech GmbH verwendet. Der Ligand ist Trimethylamin, wobei R die Matrix bezeichnet ($R-N^+-(CH_3)_3$).
Die Komponenten wurden sowohl einzeln als auch in der Kombination Q Sepharose® FF HiPrep®-Säule und Sartobind® Q Nano in eine Chromatographie-Anlage "ÄKTA Prime plus" der Firma GE Healthcare (nachfolgend "ÄKTA") eingesetzt. Das aus den Adsorbern austretende Fluid wurde in einem integrierten Durchflussphotometer bei 280 nm vermessen. Die Daten wurden an einen angeschlossenen Rechner übertragen und graphisch dargestellt.
Es wurde zunächst die Systemdispersion an Hand von Durchbruchskurven mit der nichtbindenden Substanz Aceton für verschiedene Kombinationen aufgenommen. In der Figur 1 sind die jeweiligen Durchbruchskurven dargestellt.
Die hierbei ermittelten Werte der Totvolumina werden im Folgenden in Tabelle 1 dargestellt:

Tabelle 1: Übersicht der Totvolumina

| Konfiguration | Totvolumen (ml) |
|---|---|
| ÄKTA | 5,90 |
| ÄKTA + Nano | 10,48 |
| ÄKTA + HiPrep | 22,55 |
| ÄKTA + HiPrep + Nano | 26,44 |

Es wurden nun Durchbruchskurven mit Rinderserumalbumin (RSA) aufgenommen. Dazu wurde RSA der Fa. SIGMA-Aldrich, Bestellnummer A2153, in einer Konzentration von 2 Gramm/Liter in einem 0,01 Mol/Liter-Kaliumphosphatpuffer mit pH 7,0, welcher durch Mischen von $KH_2PO_4$-Lösung mit $K_2HPO_4$-Lösung hergestellt wurde, gelöst und über Membranfiltereinheiten Sartolab RF Vakuumfiltrationseinheiten, Best. Nr. 18082-E, der Fa. Sartorius Stedim Biotech GmbH Göttingen, Deutschland, mit einer nominellen Porenweite von 0,2 μm sterilfiltriert. Mit dieser Lösung wurden dynamische Bindungskapazitäten (DBK) bei verschiedenen Geschwindigkeiten aufgenommen. Die Ergebnisse sind in den Figuren 2, 3 und 4 dargestellt.

Beispiel 2: Messung von Durchbruchskurven

Mit den in Beispiel 1 beschriebenen Komponenten, d.h. Säule und Membranadsorber, wurden vollständige Durchbruchskurven (einschließlich Waschen und Elution mit 1 M NaCl) ermittelt.

Tabelle 2: Verwendete Abkürzungen

| C = Konzentration |
|---|
| DBK = Dynamische Bindungskapazität |
| M = Menge (mg) |
| MA = Membranadsorber |
| tot = total |
| ser. = seriell |
| V = Volumen (ml) |

Es werden folgende Symbole, Begiffsbestimmungen und Formeln verwendet:

$$M_{bt,10\%} = \text{Beladung mit RSA bis 10\% Durchbruch.}$$

Von $M_{bt,\ 10\%}$, wird die Menge Protein im Totvolumen abgezogen:

$$M_{ads,10\%} = M_{bt,10\%} - C_{Ausgangslösung} \times V_{tot}$$

Die Menge des eluierten Proteins wurde durch Aufsummieren der in den Elutionsfraktionen gemessenen Mengen ermittelt.

Die Zunahme der gebundenen Menge an Protein auf Grund der seriellen Schaltung von Säule und Membranadsorber bei der jeweiligen Flussrate ist folgendermassen definiert:

Dynamische Bindungskapazität bei 10 % Durchbruch:

$$DBK_{10\%} = \frac{M_{ads,10\%}}{V_{Adsorbens}} \quad ,$$

wobei Adsorbens den jeweils verwendeten Adsorber bezeichnet.

Dynamische Bindungskapazität der Q Sepharose® FF HiPrep®-Säule bei 10 % Durchbruch für die serielle Schaltung:

$$DBK_{10\%} = \frac{M_{ads,10\%\ ser.\ Schaltung} - M_{ads,10\%\ MA}}{V_{Säule}}$$

$$\Delta M_{ads,\ 10\%} = M_{ads,\ ser.\ Schaltung,10\%} - M_{ads,\ Säule,10\%}$$

$$\Delta M_{ads,\ rel,\ 10\%} = \frac{M_{ads,ser.\ Schaltung\ 10\%} - M_{ads,\ Säule,10\%}}{M_{ads,Säule,10\%}} \times 100\%$$

Die Effizienz E des Membranadsorbers (das Verhältnis von $\Delta M_{ads,\ 10\%}$ zur Kapazität des Membranadsorbers wird folgendermassen bestimmt:

$$E = \frac{\Delta M_{ads,\ 10\%}}{M_{ads,MA,10\%}} \times 100\%$$

Tabelle 3: Zusammenfassung der erhaltenen Ergebnisse mit Membranadsorber und Säule alleine und in der seriellen Verschaltung

| | MA alleine | Säule alleine | Serielle Schaltung | | |
| --- | --- | --- | --- | --- | --- |
| Flussrate (ml/min) | $M_{ads,\ 10\%}$ (mg total) | $M_{ads,\ 10\%}$ (mg total) | $M_{ads,\ 10\%}$ (mg total) | $\Delta M_{ads,\ 10\%}$ (mg total) | $\Delta M_{ads,\ rel,\ 10\%}$ (%) | Effizienz (%) |
| 5 | 51,0 | 1297 | 1401 | 104 | 8,0 | 203 |
| 10 | 55,8 | 1167 | 1326 | 159 | 13,6 | 284 |

Tabelle 4: Produktivität der Säule alleine und der seriellen Verschaltung

| Flussrate (ml/min) | Proteinkonzentration (mg/ml) | Beladungszeit (min) | Produktivität (mg/ml x min) | |
|---|---|---|---|---|
| | | | Säule alleine | Serielle Schaltung |
| 5 | 1,9 | 137 | 0,48 | --- |
| 10 | 2,2 | 60 | --- | 0,97 |

In allen 3 untersuchten Fällen, (Figuren 2 bis 4), zeigt die Kombination eine Vergrößerung der dynamischen Bindungskapazität, d.h. der bei 10 % Durchbruch gebundenen Menge an Protein, und weiterhin einen steileren Anstieg der Durchbruchskurve, da das aus der Säule austretende Protein am Membranadsorber gebunden wird. Die Steilheit der Durchbruchskurve der erfindungsgemäßen Vorrichtung mit serieller Schaltung wird dann von der Durchbruchskurve des Membranadsorbers bestimmt. Dieses Verhalten ist bei höheren Flussgeschwindigkeiten und größeren Membranadsobereinheiten noch stärker ausgeprägt.

Aus Tabelle 3 geht hervor, dass die Effizienz des Membranadsorbers durch die serielle Verschaltung mit der Säule extrem stark (203 bis 284 %) ansteigt.

Aus Tabelle 4 geht hervor, dass die Produktivität der seriellen Schaltung gegenüber der Säule alleine ohne Verlust der dynamischen Bindungskapazität verdoppelt wurde, obwohl nur ein geringes Volumen (13 % des gesamten Adsorbervolumens) an Membranadsorber hinzugefügt wurde.

**Patentansprüche**

1.  Vorrichtung zum Trennen bzw. Isolieren von Substanzen in bzw. aus einem Gemisch in einem Fluid, welche mindestens eine diffusiv betreibbare Chromatographiematrix und mindestens eine nachgeschaltete, konvektiv betreibbare, flächenförmig ausgestaltete Chromatographiematrix umfasst, wobei die konvektiv betreibbare Chromatographiematrix der diffusiv betreibbaren Chromatographiematrix so nachgeschaltet ist, dass das die diffusiv betreibbare Chromatographiematrix verlassende Fluid durch die konvektiv betreibbare Chromatographiematrix leitbar ist, wobei die diffusiv betreibbare Chromatographiematrix eine Gelchromatographiematrix ist, und wobei die konvektiv betreibbare Chromatographiematrix ein Membranadsorber mit mindestens einer Membran ist.

2.  Vorrichtung nach Anspruch 1, wobei das die diffusiv betreibbare Chromatographiematrix verlassende Fluid vollständig durch die konvektiv betreibbare Chromatographiematrix leitbar ist.

3.  Vorrichtung nach einem der vorhergehenden Ansprüche, wobei die diffusiv betreibbare und/oder die konvektiv betreibbare Chromatographiematrix mindestens einen Liganden umfaßt, der über mindestens eine chemische und/oder physikalische Wechselwirkung mit mindestens einer Substanz in dem Gemisch interagiert.

4.  Vorrichtung nach Anspruch 3, wobei die diffusiv betreibbare und die konvektiv betreibbare Chromatographiematrix mindestens einen gleichen Liganden umfassen.

5.  Vorrichtung nach Anspruch 3 oder 4, wobei der Ligand aus der Gruppe der Ionenaustauscher, salztoleranten Liganden, Chelatbildner, thiophilen oder hydrophoben Liganden verschiedener Kettenlängen und Konfigurationen, "Reversed-Phase"-Liganden, reaktiven und anderen Farbstoffen, der anorganischen Moleküle und Ionen, deren organischen und anorganischen Verbindungen, Affinitätsliganden, hochmolekularen Liganden, Enzyme und Untereinheiten sowie Teilen davon, Strukturproteine, Rezeptoren und Effektoren sowie Teilen davon, Viren und Teilen davon, Xenobiotika, Pharmazeutika und pharmazeutischen Wirkstoffe, Alkaloide, Antibiotika, Biomimetika und der Katalysatoren ausgewählt ist.

6.  Verfahren zum Trennen bzw. Isolieren von Substanzen in bzw. aus einem Gemisch, umfassend die Schritte:

    (a) gegebenenfalls Auflösen des Gemisches in einem geeigneten Lösungsmittel oder Lösungsmittelgemisch, um ein Fluid zu erhalten,
    (b) Hindurchleiten des Fluids durch die Vorrichtung nach einem der Ansprüche 1 bis 5, und
    (c) Sammeln des hindurchgeleiteten Fluids.

7.  Verfahren nach Anspruch 6, wobei das Gemisch eine Lösung aus einer Synthese, ein biomolekülhaltiges Fluid oder ein biopartikelhaltiges Fluid ist.

**8.** Verwendung der Vorrichtung nach einem der Ansprüche 1 bis 5 zum Trennen bzw. Isolieren einer Zielsubstanz in bzw. aus einem Gemisch.

## Claims

**1.** An apparatus for separating or isolating substances in or from a mixture in a fluid, comprising at least one diffusion-based chromatography matrix and at least one downstream convection-based planar chromatography matrix, wherein the convection-based chromatography matrix is downstream of the diffusion-based chromatography matrix such that the fluid leaving the diffusion-based chromatography matrix can be conducted through the convection-based chromatography matrix,
wherein the diffusion-based chromatography matrix is a gel chromatography matrix, and
wherein the convection-based chromatography matrix is a membrane adsorber having at least one membrane.

**2.** The apparatus according to claim 1, wherein the fluid leaving the diffusion-based chromatography matrix can be conducted completely through the convection-based chromatography matrix.

**3.** The apparatus according to any of the preceding claims, wherein the diffusion-based chromatography matrix and/or the convection-based chromatography matrix comprises at least one ligand which interacts with at least one substance in the mixture via at least one chemical and/or physical interaction.

**4.** The apparatus according to claim 3, wherein the diffusion-based chromatography matrix and the convection-based chromatography matrix comprise at least one identical ligand.

**5.** The apparatus according to claim 3 or 4, wherein the ligand is selected from the group of the ion exchangers, salt-tolerant ligands, chelating agents, thiophilic or hydrophobic ligands of varying chain lengths and configurations, reversed-phase ligands, reactive dyes and other dyes, the inorganic molecules and ions, organic and inorganic compounds thereof, affinity ligands, high molecular weight ligands, enzymes and subunits and parts thereof, structural proteins, receptors and effectors and parts thereof, viruses and parts thereof, xenobiotics, pharmaceuticals and pharmaceutical active ingredients, alkaloids, antibiotics, biomimetics and the catalysts.

**6.** A method of separating or isolating substances in or from a mixture, comprising the steps of:

(a) optionally dissolving the mixture in a suitable solvent or solvent mixture in order to obtain a fluid,
(b) conducting the fluid through the apparatus according to any one of claims 1 to 5, and
(c) collecting the conducted fluid.

**7.** The method according to claim 6, wherein the mixture is a solution from a synthesis, a biomolecule-containing fluid or a bioparticle-containing fluid.

**8.** Use of the apparatus according to any one of claims 1 to 5 for separating or isolating a target substance in or from a mixture.

## Revendications

**1.** Dispositif pour la séparation ou la purification de substances dans ou à partir d'un mélange dans un fluide, ledit dispositif comportant au moins une matrice chromatographique opérable par diffusion et au moins une matrice chromatographique de surface agencée en aval, opérable par convection, la matrice chromatographique opérable par convection étant montée en aval de la matrice chromatographique opérable par diffusion de telle sorte que le fluide quittant la matrice opérable par diffusion est mené au travers de la matrice chromatographique opérable par convection, la matrice chromatographique opérable par diffusion étant une matrice de chromatographie sur gel et la matrice chromatographique opérable par convection étant un adsorbeur à membrane comportant au moins une membrane.

**2.** Dispositif selon la revendication 1 dans lequel le fluide quittant la matrice opérable par diffusion est mené dans sa totalité au travers de la matrice chromatographique opérable par convection.

**3.** Dispositif selon l'une des revendications précédentes dans lequel la matrice chromatographique opérable par diffusion et/ou la matrice chromatographique opérable par convection comporte au moins un ligand qui interagit moyennant au moins une interaction chimique et/ou physique avec au moins une substance dans le mélange.

**4.** Dispositif selon la revendication 3 dans lequel la matrice chromatographique opérable par diffusion et la matrice chromatographique opérable par convection comportent au moins un même ligand.

**5.** Dispositif selon la revendication 3 ou 4 dans lequel le ligand est choisi parmi le groupe comportant les échangeurs d'ions, les ligands tolérants au sel, les agents de chélation, les ligands thiophiles ou hydrophobes de diverses longueurs de chaîne et de diverses configurations, les ligands « reversed-phase », les colorants réactifs et autres colorants, les molécules et ions inorganiques, leurs composés organiques et inorganiques, les ligands par affinité, les ligands de poids moléculaire élevé, les enzymes et sous-unités et fractions de celles-ci, les protéines structurelles, les récepteurs et effecteurs ainsi que les parties de ceux-ci, les virus et parties de ceux-ci, les composés xénobiotiques, les composés pharmaceutiques et les principes actifs pharmaceutiques, les alcaloïdes, les antibiotiques, les composés biomimétiques et les catalyseurs.

**6.** Procédé pour la séparation ou la purification de substances dans ou à partir d'un mélange, comportant les étapes :

(a) éventuellement dissolution du mélange dans un solvant ou mélange de solvants adéquats pour la préparation d'un fluide,
(b) le passage du fluide au travers du dispositif selon l'une des revendications 1 à 5, et
(c) collecte du fluide qui a traversé ledit dispositif.

**7.** Procédé selon la revendication 6 dans lequel le mélange est une solution d'une synthèse, un fluide contenant des biomolécules ou un fluide contenant des bioparticules.

**8.** Utilisation du dispositif selon l'une des revendications 1 à 5 pour la séparation ou la purification d'une substance cible dans ou à partir d'un mélange.

Figur 1

Figur 2

Figur 3

Figur 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6294090 B1
- DE 19711083 A1
- WO 03051483 A1
- US 20070241061 A1

**In der Beschreibung aufgeführte Nicht-Patentliteratur**

- **HAHN et al.** *J. Chromatogr. A,* 2005, vol. 1093, 98-110